# EUROPEAN PATENT APPLICATION

(11) **EP 4 641 172 A1**
(43) Date of publication of application: **29.10.2025**
(21) Application number: 24173564.6
(22) Date of filing: 30.04.2024
(51) Int. Cl.: G01N 21/64, G01N 21/77, G01N 21/94, G01N 33/28

(54) **MULTI-LAYER OPTICAL SENSOR FOR DETECTING PESTICIDES IN OIL AND DETECTOR DEVICE COMPRISING THE SAME**

(30) Priority: 26.04.2024 PT 2024119433
(71) Applicant: Universidade de Évora, 7000-803 Évora (PT); UNIVERSIDADE NOVA DE LISBOA, 1099-085 Lisboa (PT)
(72) Inventor: GARCIA, RAQUEL MARTA NEVES DOS SANTOS, 7005-482 ÉVORA (PT); CABRITA, MARIA JOÃO PIRES DE BASTOS, 7000-706 ÉVORA (PT); CARREIRO, ELISABETE DA PALMA, 7750-381 MÉRTOLA (PT); RAMALHO, JOÃO PAULO, 7005-351 ÉVORA (PT); IGREJA, RUI ALBERTO, 2820-670 CHARNECA CAPARICA (PT); ÁGUAS, HUGO MANUEL BRITO, 2820-524 CHARNECA DA CAPARICA (PT)
(74) Representative: Monteiro Alves, Inês

(57) **Abstract**

The present invention refers to a multi-layer optical sensor for detecting pesticides in oil and to a detector device comprising the same.

The optical sensor of the invention comprises a substrate layer (10), a photosensitive layer (11), an electrode layer (12), an optical filter layer (13), a sensitive layer (14), wherein the sensitive layer (14) has fluorescent properties, includes at least one molecularly imprinted polymer and the photosensitive layer (11) absorbs incident light (100).

## Description

### TECHNICAL FIELD OF THE INVENTION

This disclosure relates to the field of sensors for detecting traces of pesticides in olive oil. In particular, the present invention relates to optical sensors including Molecularly Imprinted Polymers (MIPs) with a fluorescent active element embedded therein.

### BACKGROUND OF THE INVENTION

Plant protection products - commonly called pesticides - are used in olive groves to prevent plagues and diseases. Improper use or disregard for the safety withdrawal period may cause their persistence until the olives are harvested. Given that olive oil is obtained from olive milling merely through physical and/or mechanical processes, the presence of these chemical contaminants in the oil may occur. Given the harmful effect of these contaminants, it is imperative that the level of these products is properly controlled. The reference methods are based on chromatographic techniques - High Performance Liquid Chromatography (HPLC) and Gas Chromatography (GC) associated to mass spectrometry (MS). Despite having high sensitivity, these techniques are expensive, time-consuming, and require skilled technicians. Recently, new sensor-based approaches have been explored, aiming to overcome the drawbacks of conventional methodologies. However, the development of sensors for the detection of pesticides in olive oil focuses fundamentally on enzymatic biosensors. Acetylcholinesterase (AChE) is one of the most investigated due to the inhibitory effect that organophosphate pesticides (OP) exert on enzymatic activity. Furthermore, it provides quantitative information with high precision, low cost and short response time, however it has disadvantages - enzymatic instability, matrix interference with AChE and irreversibility of the AChE-OP interaction, which prevent its widespread use and commercialization. Furthermore, selectivity remains a challenge to overcome.

### PRIOR ART

Patent application US2021179798A1 entitled "Polymer Based Sensors for Detecting Agricultural Analytes and Methods of Making Same", from GEORGIA TECH RES INST [US], published on 2021-06-17 discloses a polymer-based sensor for detecting agricultural analytes, including stable polymer-based sensing films such as molecular imprinted polymers (MIPs) that can be incorporated in sensors for detecting herbicides and pesticides, as well as methods of making the sensing films.

Patent application EP2012050899W entitled "FIBRE-OPTIC SENSOR", from UNIV CITY [GB]; NGUYEN HIEN [GB]; SUN TONG [GB]; GRATTAN KEN [GB], published as WO2012098241A2 on 2012-07-26 discloses an optic fibre sensor comprising an optic fibre (9) and a molecularly imprinted polymer (MIP) receptor (10) formed directly on said fibre (9), wherein: said polymer includes a fluorophore that fluoresces when exposed to a source of light, said MIP is selective for a particular drug of interest, and said fluorescence changes when said MIP is exposed to said drug of interest.

None of the prior-art documents presents a solution to
address the above-mentioned problems.

### SUMMARY OF THE INVENTION

The present invention pertains to a multi-layer optical sensor with fluorescent properties using MIPs as recognition elements associated with fluorescence for signalling detection of the pesticide residues is developed. This sensor includes:
i) the use of molecular imprinting technology for the generation of artificial receptors (MIPs) that possess chemically selective binding sites able to recognize the target molecule;
ii) the active sensing element is directly integrated into the MIP, then the fluorescence modulation allows to signalling the presence and concentration of the target analyte.

By fluorescence modulation it is meant the following:
the fluorescence intensity will suffer a quenching after the reaction of the target pesticide and MIP.

In certain embodiments, the present invention refers to a multi-layer optical sensor for detecting pesticides in oil, comprising the following layers: a substrate layer (10), a photosensitive layer (11), an electrode layer (12), an optical filter layer (13), a sensitive layer (14) comprising MIPs and fluorescent properties.

In other embodiments of the present invention, the multi-layer optical sensor for detecting pesticides comprises additionally a first dielectric layer (131) and a second dielectric layer (132).

In yet other embodiments of the present invention, the optical filter layer (13) comprises a multilayer stack of dielectric layers.

In certain embodiments of the present invention, the fluorescent properties of the sensitive layer (14) are provided by fluorescent particles.

In a first aspect, the invention relates to a multi-layer optical sensor for detecting pesticides in oil.

In a second aspect, the invention relates to a detector device for detecting pesticides in oil.

### TECHNICAL PROBLEM

The technical problem to be solved by the present solution can be formulated as: how to create a detector of pesticides (such as, organophosphorus, pyrethroids and triazines classes) in olive oil which associates a high selectivity in the pesticide traces detection with portability and easy of use, being at the same time robust, chemically stable, low cost and reusable.

### SOLUTION TO TECHNICAL PROBLEM

The present invention provides an optical multi-target analysis sensor device that will allow the simultaneous quantification of pesticides from different classes that are approved for application in olive groves, which represents a significant advance in the area of pesticide analysis. The invention includes an innovative sensor (MSC "Mix Sensor Chip"). This type of sensor brings together the following features:
i) the use of molecular printing technology to create artificial receptors (MIPs) that have selective binding sites capable of recognizing the target molecule;
ii) an active detection element is directly integrated into the MIP, so that fluorescence allows signalling the presence and concentration of the target analyte. In detail, the sensor comprises a double layer of, for example, hydrogenated amorphous silicon (a-Si:H) and amorphous silicon carbide (a-SiC:H) that are manufactured by plasma-enhanced chemical vapor deposition (PECVD) and photolithographic process. The deposition of the MIP layer is carried out by "spin coating", allowing precise control of the layer thickness, allowing the response time and sensitivity of the sensor to be adjusted. The sensitive layer thickness may modify either the response time or the sensor sensitivity, however, thickness isn't the only parameter. A same thickness may have more concentration, or less, of the fluorescent element. For a same concentration of the fluorescent element per unit volume, the thickness can be changed in order to obtain higher sensitivity or shorter response time, for example. In particular, a proper control of the sensitive layer thickness enables a proper control of the response time and sensitivity.

### ADVANTAGEOUS EFFECTS OF THE INVENTION

The technological solution provided by the present invention has the advantage of combining high selectivity in the trace detection of these contaminants with portability. This combination allows the introduction of an analytical tool, easy to use and without the need for expensive instrumentation and specialized technicians to handle it. This technological solution also combines robustness and chemical stability, low cost and the possibility of reuse. Furthermore, it can be used throughout the production process, to determine the levels of these compounds, from the mill to packaging and marketing.

These and other features and advantages of the invention will become apparent in the detailed description together with the following drawings.

### BRIEF DESCRIPTION OF THE FIGURES

Preferred embodiments of the present invention will be described with reference to the accompanying figures, which are to be construed as non-limiting the invention scope, which scope is defined by the appended claims.

Any subsequent changes or modifications of the inventive features illustrated herein, as well as any additional applications of the illustrated principles and embodiments of the invention, which would normally occur to a person skilled in the art in possession of this description, are considered to be within the scope of the claimed invention.
**Figure 1** - schematically represents the layers of a first preferred embodiment of a multi-layer optical sensor according to the invention;
**Figure 2** **-** schematically represents the MIP synthetic procedure, which is not part of the present invention; and
**Figure 3** - schematically represents the layers of a second embodiment of a multi-layer optical sensor according to the invention.

### DESCRIPTION OF EMBODIMENTS

The multi-layer sensors of the present invention are designed to be dipped in olive oil, or in any other oil, to detect traces of a pesticide that could be harmful for human health. Olive oil presents a high viscosity. So, a diluted solution of olive oil in apolar solvents (n-heptane, n-hexane) can be used on the experimental determination with the sensor. The sensor will be dipped in the diluted solution of the olive oil.

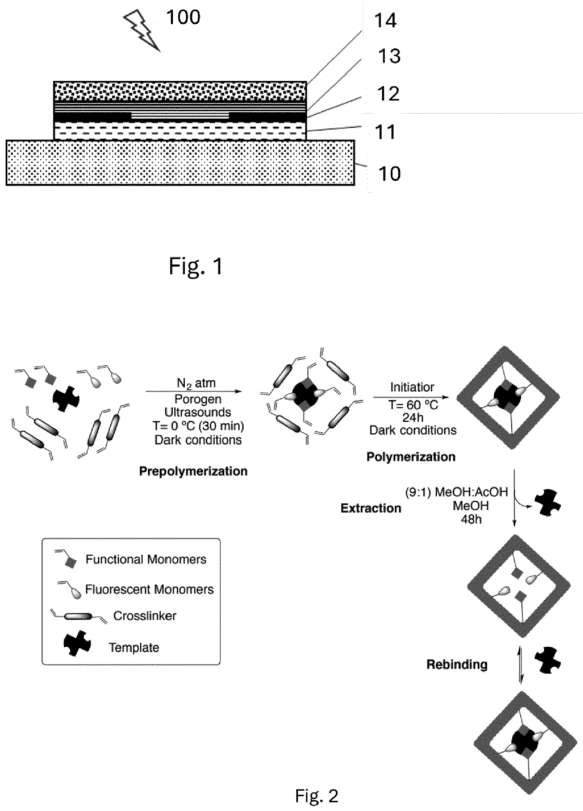

The multi-layer sensors of the present invention have the shape of a thin film and may have a different number of layers depending on the electrical properties of the layer used as a filter. In a preferred first embodiment, the optical filter layer (13) is a dielectric layer and the multi-layer optical sensor according to said preferred first embodiment comprises five layers, as shown in **Figure 1** and hereinafter described.

The first layer is a substrate layer (10) upon which a photosensitive layer (11) is deposited. The substrate layer (10) maybe be rigid, for example, glass-based, Si-based or any type of flexible substrate such as polyamide, PEN (PEN, stands for Polyethylene naphthalate, Kapton, among any other polymer which is stable at the temperatures of operation and inert in the processes used for the deposition and patterning of the layers, or the substrate is a combination of any of these materials. The photosensitive layer (11) is deposited upon the substrate layer (10) through a deposition process known to a skilled person in the art. For example, several thin film deposition techniques, depending on the semiconductor material, can be employed for its deposition. In the case of amorphous silicon, the most common one is Plasma Enhanced Chemical Vapour Deposition (PECVD). The photosensitive layer (11) is a layer which properties change with light. For example, the photosensitive layer (11) may be a photoresistive layer, wherein in such layer the electric resistance varies with light, or optical radiation incident on it.

The photosensitive layer (11) will be sensitive to incident light (100) represented in Figure 1 by a flash sign. The light (100) is absorbed by the photosensitive layer (11). In fluorescent sensors, MIPs only act as the sensitive element of the sensor, since incorporates fluorescent monomers. Indeed, fluorescent monomers present in the MIP structure will interact with the target pesticide resulting in a quenching in the fluorescence intensity, being applied as the signal readout of the sensor. The fluorescent MIPs offer a detection mechanism that reports chemical recognition events.

Any photosensitive material can be used in the photosensitive layer (11), being a referred choice the amorphous silicon (a:Si), that is, the hydrogenated amorphous silicon (a-Si:H). Any other semiconductor material with a high level of photosensitivity can be used. The amorphous silicon a:Si has a high photosensitive level and it involves a low cost, is easy to produce compared to other inorganic semiconductors. There are organic semiconductors that are cheaper and also easy to deposit, but these semiconductors are less stable than the inorganic ones. Other possible materials can be found in the reference "Silicon Photo Multipliers Detectors Operating in Geiger Regime: an Unlimited Device for Future Applications" (Scientific Figure on ResearchGate).

Still in reference to Figure 1, the next layer is an electrode layer (12). Here, there are electrodes placed upon the photosensitive layer (11). The electrodes (two or more) used in the electrode layer (12) are made of a conductive material, for example, any metal, a conductive polymer, ITO (where ITO stands for Indium Tin Oxide), among others. At least, the electrode layer comprises two electrodes of the same type or of different types.

The metal electrodes can be deposited by any Physical Vapour Deposition (PVD) technique, such as evaporation in vacuum or sputtering.

The two or more electrodes in the electrode layer measure the electric resistance. Depending on the electrode's geometry, the nominal value of the sensor resistance is influenced and defines the sensor model.

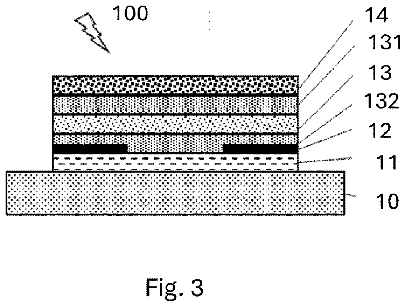

On top of the electrode layer (12) an optical filter layer (13) is provided. This optical filter layer (13) acts as a filter to reduce the intensity of the exiting light reaching the sensor, that is the sensitive layer (14) described hereinafter and the optical filter layer (13) can be made of a material able to absorb or scatter the exciting radiation. The dielectric optical filter layer (13) can also be deposited by several PVD and CVD techniques, such as Sputtering, Atomic Layer Deposition (ALD) and electron-beam, to name a few. For example, semiconductors with adequate bandgap or metal nanoparticles are good examples. As a proof-of-concept Ag (silver) nanoparticles with tailored size were used to have their absorption optical band tuned with the wavelength of the exciting radiation, that is, the incident light which is partially absorbed by the fluorescent element, as it will be discussed in reference to Figure 3. As yet shown in Figure 1, the optical filter layer (13) extends down to the electrode layer (12) in order to fill in the spaces between the electrodes and thereby provide electric insulation between said electrodes.

The sensitive layer (14) shown in Figure 1 comprises one or more molecularly imprinted polymers (MIPs). MIPs are synthetic polymers with a predetermined selectivity for a given analyte. MIPs are synthetized in the laboratory, through a chemical reaction that produces MIPs. In this synthetic procedure schematically shown in Figure 2 several reagents (crosslinker, functional monomer, fluorescent monomer, template, radical initiator, porogen (solvents)) are used, enabling the occurrence of a polymerization reaction. The synthetic procedure details the molar ration between the reagents, the temperature and all the conditions used with the synthesis. This synthesis of MIPs includes the use of a crosslinking agent, a functionalized monomer, a fluorescent monomer, such as fluorescein-derivatives, coumarin-derivatives, series of pyrene-based compounds, porphyrin-based systems, the template molecule and a porogen, enabling the production of a three-dimensional structure with cavities complementary in size and shape, to the target molecule (template). Thus, MIPs are tailor made polymers, being highly selective materials that mimic the shape-specific binding mechanism, which is found naturally occurring in the biological structures. Thus, MIPs are also called as "artificial receptors". For example, the synthetic procedure schematically shown in Figure 2 represents the production of MIPs selective for the recognition of deltamethrin and terbuthylazine (pesticides) in olive oil. Other pesticides can be selectively detected with the sensor of the invention since MIPs can be considered a tailor-made product for the detection of a pre-defined pesticide. MIPs can recognize several classes of pesticides - triazines, pyrethroids, organophosphorus, among others. For that, the following modification in the production procedure has to be done: molar ratio between the functional monomer and the fluorescent monomer. In particular, Figure 2 represents a MIP synthetic procedure, wherein the functional monomer is an acrylamide; the fluorescence monomer is a fluorescein derivative and coumarin derivatives; the crosslinker is EGDMA (Ethylene glycol dimethylacrylate); the template of the target molecule is deltamethrin (Dm) and terbuthylazine (Tbz)). Those target molecules have been selected since they are used as pesticides to prevent pests and diseases in olive orchards. If the withdraw interval since the pesticide application is not respected, the residue of these pesticides could be present in olive oil. The development of such a sensing tool for trace analysis of pesticides in olive oil is highly advantageous, since detecting pesticides in oil is a food safety problem. Such a sensor with higher sensitivity due to fluorescence, provides a powerful tool for the safe detection of pesticide traces in olive oil.

**Figure 3** schematically represents the layers of a second embodiment of a multi-layer optical sensor. In this second embodiment, the optical filter layer (13) comprises conductive particles, even though they do not form a conductive film due to the fact that the particles are separated from each other. For example, nanoparticles of silver (Ag Nps).

As for the Ag NPs, the most common deposition method is the dewetting method, which consists in the evaporation of Ag in vacuum, with a small thickness (for example, less than 10 nm) while the substrate is heated at a temperature above 80°C. Ag nanoparticles then formed on the surface and present a plasmonic absorption band that can be tuned to certain wavelength as function of the NPs size. The plasmonic absorption band acts as the optical filter of the excitation radiation, that is the incident radiation (100).

The main advantage of the usage of the Ag NPs is the ability to adjust the optical filter wavelength produced thereby, obtaining a light intensity attenuation for a specific wavelength that matches the extinction frequency of the Ag NPs. This extinction frequency, that depends on the size of the Ag NPs, should be engineered to match the exciting frequency, preventing it from reaching the sensor, while allowing the emitting frequency to pass through them without attenuation, reaching the sensor. The extinction band of the Ag NPs, in this case, is narrow enough to allow the discrimination of the exciting and emitting wavelengths, despite of being very close to each other.

In this second embodiment, two extra layers are needed in order to electrically insulate the optical filter conductive layer (13) from the electrodes layer (12) and from the sensitive layer (14). As Figure 3 shows, a first dielectric layer (131) is provided between the sensitive layer (14) comprising the MIPs and the optical filter layer (13) and a second dielectric layer (132) is provided between the optical filter layer (13) and the photosensitive layer (11) and electrode layer (12).

For the first dielectric layer (131) and the second dielectric layer (132), any transparent electrically insulating material can be used. Examples of possible materials that can thereon be applied are TiO₂, SiO₂, SiN, among others. The second dielectric layer (132) has the function to electrically insulate the photosensitive layer (11) and electrode layer (12) from the optical filter layer (13) comprising, for example, Ag NPs. The first dielectric layer (131) has the function to stabilize the optical filter layer (13), for example, comprising Ag NPs, acting as a protection layer, preventing the interaction of the MIP with the optical filter layer, avoiding any possible chemical interaction.

The optical filter layer (13) can comprise, for example, semiconductor or photonic materials, such as Ag NPs. A layer of Ag NPs is not considered conductive, since due to their size, Ag NPs present plasmonic effect. For this plasmonic effect to occur, the Ag NPs do not touch each other. They are separated. Therefore, there is no conduction. Ag NPs are selected because their plasmonic absorption band can be tuned with that of the exciting wavelength.

Typically, as an example, the first and the second dielectric layers (131,132) can have a thickness ranging between 100 and 300 nm.

In a third embodiment of the multi-layer sensor according to the invention, the optical filter layer (13) is made of a semiconductor material which can be organic, inorganic or hybrid. In the third embodiment of the sensor according to the invention, there are no dielectric layers as in the second embodiment.

In the third embodiment, a semiconductor material is selected for the optically active layer, that is, the optical filter layer (13). Instead of Ag NPs, as in the second embodiment, the semiconductor material has its optical band gap slightly below the exciting radiation wavelength of the incident light, to cut this radiation, while allowing the light emitted from the fluorescent MIP to pass through. For this specific MIP, it is difficult to find a plain semiconductor capable of performing this task. A way of surpassing this difficulty is to take a wide bandgap semiconductor like TiO₂, add doping elements such as Al, Cu, Mo or W to decrease the TiO₂ bandgap and to make it more aligned with the desired cut off wavelength. Other wide bandgap semiconductors from the realm of compound semiconductors such as ZnS are also good candidates.

The remaining layers of the third embodiment are the same as for the first embodiment, such that they will not be described again.

In a fourth embodiment of the sensor according to the invention, the optical filter layer (13) comprises a multilayer stack of dielectric layers with different refractive indexes, to cause a destructive interference for a certain wavelength, like a conventional optical filter.

The remaining layers of the fourth embodiment are the same as for the first embodiment, such that they will not be described again.

In a fifth embodiment of the present invention, the multi-layer sensor is able to simultaneously detect two different pesticide traces. For that, the following differences are described, for the layers of this fifth embodiment. In the fifth embodiment of the invention, the MIP layer, that is, the sensitive layer (14) comprises two layers having two fluorescent MIPs. These different fluorescent MIPs are able to recognize different target pesticides. The fluorescent MIPs will differ in the fluorescent monomers and also the target pesticides, in order to have recognition of two different chemical events.

Also, in each pair of electrodes a different MIP is deposited. This will cause the possibility of detecting two different analytes, i.e., two different types of pesticides in the same oil sample.

In all embodiments, the photosensitive layer (11) comprises a semiconductor, for example, amorphous silicon (a:Si). The resistivity of this photosensitive layer (11) changes significantly with the incident light (100) intensity for energies above its bandgap, leading to a variation in resistance measured on the electrodes of the electrode layer (12) deposited on top of the photosensitive layer (11), as explained above. The sensitive layer (14) comprising the MIPs, when excited with low wavelength radiation originated from the incident light (100), causes a change in the fluorescence emission intensity at higher wavelengths. The fluorescent layer absorbs light at a shorter wavelength (higher energy) and emits at a higher wavelength (lower energy). What changes here is the intensity of the emission which is modified when the analyte binds creating the emission quenching. The optical filter layer (13) attenuates the excitation radiation, allowing only the wavelengths corresponding to emission to pass through, thus altering the electrical resistivity of the photosensitive layer (11). Other suitable photosensitive material can be used in this photosensitive layer (11).

In a second aspect, the invention refers to a detector device, wherein the detector device comprises at least one multi-layer optical sensor according to the first aspect of the invention and a handle for a user to dip the detector device in the oil. With only a single analysis, the device according to the second aspect of the invention will enable the detection and quantification of target pesticides from two different classes, yet keeping enhancements in both selectivity and stability, and moreover robustness, portability and easy handling.

Certainly, this invention is by no means restricted to the embodiments described in this document, and a person skilled in the art can envision many possibilities for modifying it and replacing technical features with equivalent ones, depending on the requirements of each situation, as defined in the appended claims.

### DEFINITIONS

Throughout this application, the term "bandgap" means the distance between the valence band of electrons and the conductive band, essentially, it represents the minimum energy required to excite an electron up to a state in the conduction band where it can participate in conduction.

As used in this application, the term "or" is to be interpreted in an inclusive meaning instead of the exclusive meaning, unless otherwise clearly stated. That is, an expression like "X utilizes A or B" shall be interpreted as including all possible combinations, i.e., "X utilizes A", "X utilizes B", and "X utilizes A and B".

As used in this application, the indefinite article "a", "an", shall be interpreted as including "one" or "one or more", unless otherwise clearly stated.

Throughout this application, the examples provided shall be interpreted as having the purpose to illustrate one or more examples of embodiments of the present invention and shall not be interpreted as preferences, unless otherwise clearly stated.

As used throughout the present application, the terms "comprise/comprises", "comprising", "include/includes", "including" specify the presence of the features, elements, components, steps, and related operations, and do not exclude whatsoever the presence of further features, elements, components, steps, and related operations.

The subject-matter above-described is provided as an illustration of the present invention and shall not be interpreted as limiting it. The terminology used with the purpose of describing specific embodiments according to the present invention, shall not be interpreted as a limitation of the invention.

All changes and modifications, as long as they do not modify the essential characteristics of the following claims, must be considered within the scope of protection of the present invention.

### REFERENCE SIGNS LIST

- 10 -: substrate layer
- 11 -: photosensitive layer
- 12 -: electrode layer
- 13 -: optical filter layer
- 131 -: dielectric layer
- 132 -: dielectric layer
- 14 -: sensitive layer
- 100 -: incident light

### CITATIONS LIST

### PATENT LITERATURE

Patent application US2021179798A1 entitled "Polymer Based Sensors for Detecting Agricultural Analytes and Methods of Making Same", from GEORGIA TECH RES INST [US], published on 2021-06-17.
Patent application EP2012050899W entitled "FIBRE-OPTIC SENSOR", from UNIV CITY [GB]; NGUYEN HIEN [GB]; SUN TONG [GB]; GRATTAN KEN [GB], published as WO2012098241A2 on 2012-07-26.

### NON-PATENT LITERATURE

"Silicon Photo Multipliers Detectors Operating in Geiger Regime: an Unlimited Device for Future Applications" (Scientific Figure on ResearchGate).

## Claims

1. A multi-layer optical sensor for detecting pesticides in oil **characterized in that** it comprises the following layers:
• a substrate layer (10);
• a photosensitive layer (11);
• an electrode layer (12);
• an optical filter layer (13);
• a sensitive layer (14);
wherein
the photosensitive layer (11) is deposited on the substrate layer (10);
the electrode layer (12) is deposited on the sensitive layer (11);
the optical filter layer (13) is deposited on the electrode layer (12);
the sensitive layer (14) has fluorescent properties, includes at least one molecularly imprinted polymer, and is deposited on the optical filter layer (13); and wherein
said optical filter layer (13) is based on a material selected from the group consisting of: dielectric material, semiconductor material, photonic material, material comprising conductive particles, multilayer stack of dielectric layers with different refractive indexes; and such semiconductor material is selected from the group consisting of organic, hybrid and inorganic.

2. The multi-layer optical sensor according to claim 1 **characterized in that** it comprises the following additional layers:
• a first dielectric layer (131);
• a second dielectric layer (132);
wherein
the first dielectric layer (131) and the second dielectric layer (132) are placed on each side of the optical filter layer (13), being the first dielectric layer (131) deposited on the optical filter layer (13) and the second dielectric layer (132) deposited on the electrode layer (12), said optical filter layer (13) comprising conductive particles or comprising a photonic material, and such that said optical filter layer (13) is insulated both from electrode layer (12) and sensitive layer (14).

3. The multi-layer optical sensor according to claim 1 **characterized in that** the optical filter layer (13) comprises a multilayer stack of dielectric layers with different refractive indexes.

4. The multi-layer optical sensor according to claim 1 **characterized in that** the fluorescent properties of the sensitive layer (14) are provided by particles selected from the group consisting of: acrylamide, fluorescein-based particle, coumarin-based particle, series of pyrene-based compounds, porphyrin-based systems, and any combination of these.

5. The multi-layer optical sensor according to claim 1 **characterized in that** when two or more different types of molecularly imprinted polymers are comprised in the sensitive layer (14), the electrode layer (12) comprises a different molecularly imprinted polymer in each pair of electrodes.

6. A detector device for detecting pesticides **characterized in that** it comprises the following:
- at least one multi-layer optical sensor as defined in any one of the previous claims;
- a handle removably connected to the at least one multi-layer optical sensor;
wherein the detector device is configured to detect pesticides in an oil when dipped in it.
